# EUROPÄISCHE PATENTANMELDUNG

(11) **EP 2 497 503 A1**
(43) Veröffentlichungstag der Anmeldung: **12.09.2012**
(21) Anmeldenummer: 12170839.0
(22) Anmeldetag: 01.08.2008
(51) Int. Cl.: A61L 15/60, A61L 15/44

(54) **Blutstillender Wundpflegeartikel**

(30) Priorität: 03.08.2007 DE 102007036755
(62) Teilanmeldung aus: 08786782.6
(71) Anmelder: Riesinger, Birgit, 48149 Münster (DE)
(72) Erfinder: Riesinger, Birgit, 48149 Münster (DE)
(74) Vertreter: Storz, Ulrich

(57) **Zusammenfassung**

Gegenstand der Erfindung ist ein Wundpflegeartikel, aufweisend eine Menge an superabsorbierenden Polymeren. Der Wundpflegeartikel ist dadurch gekennzeichnet, dass er mindestens ein Agenz aufweist, das die Blutung oder die Blutungsneigung einschränken kann. Dabei kann es sich um einen chemisch und/oder pharmakologisch und/oder physiologisch wirkenden Wirkstoff bzw. Wirkstoffkomplex und/oder um ein physikalisch wirkendes Wirkelement handeln (Fig. 5).

## Beschreibung

Die vorliegende Erfindung betrifft einen Wundpflegeartikel aufweisend eine Menge an superabsorbierenden Polymeren gemäß dem Oberbegriff des Anspruchs 1.

### Stand der Technik

Flächige Wundauflagen sind längstens bekannt. Auch flächige Wundauflagen, die superabsorbierende Polymere enthalten, sind bekannt. Bei solchen Wundauflagen kann es sich z.B. um eine Wundauflage zur Absorption von Wundexsudaten handeln, wie sie z.B. in der DE10059439 sowie der WO03094813 der Anmelderin der vorliegenden Erfindung beschrieben ist, deren Inhalt vollumfänglich dem Offenbarungsgehalt der vorliegenden Beschreibung hinzugefugt werden soll.

Die genannten Wundauflagen dienen jedoch im Wesentlichen zu Zwecken des Debridements, der Kontrolle von überschüssiger Exsudation sowie dem mechanischen Schutz der Wundregion. Die zusätzliche Beimengung von Stoffen, die koagulierend, blutungsstillend, hämostyptisch oder ähnlich wirken, ist bisher unbekannt.

Dies kann jedoch von großer Bedeutung sein, da gerade akute Wunden oftmals starke Blutungen aufweisen und deren Heilung durch die o.g. Eigenschaften der bekannten Stoffe begünstigt wird. Dies gilt auch für chronische Wunden, die auf dem Boden von Läsionen bei Verbandwechseln z.B. zur freien Blutung neigen können.

Solche Fälle können sich bei Unfallpatienten, Amputationen, iatrogenen Wunden oder Dehiszenzen ereignen. Denn die Blutung erzeugt nicht nur eine Gefahrdung für den betroffenen Patienten, sondern bildet auch, gerade am Unfallort, eine nahezu unkontrollierbare Infektionsquelle für Helfer und andere Betroffene. Hinzu kommt, dass eine nicht kontrollierte Blutung Ursache für einen Schock oder - in minder schweren Fällen - für eine erhebliche psychische Krise des Patienten sein kann, der bzw. die seine Gesamtkonstitution bzw. seine Überlebenschancen erheblich beeinträchtigt.

In der Akutversorgung blutender Wunden wird nach wie vor vornehmlich auf die physikalische Blutstillung gesetzt. Hier kommen insbesondere das Abbinden und der Druckverband zum Einsatz.

Die WO99/59647 beschreibt einen vielschichtigen blutstillenden Verband, der vorzugsweise eine Thrombinschicht zwischen zwei Fibrinogenschichten enthält. Der Verband kann andere Materialien wie Glykolsäure oder auf Säure basierende Milchpolymere oder Copolymere enthalten.

Ein blutstillender Verband wird auch in WO/97/28832 beschrieben, und weist ebenfalls Thrombin sowie Fibrinogen in einer faserartigen Matrix auf.

Thrombin und Fibrinogen sind körpereigene Stoffe, die z.B. aus Blutspenden isoliert werden. Ihre Verwendung kann serologische und immunologische Probleme aufwerfen sowie Infektionen verursachen.

Aus der US5800372 ist eine Wundauflage aufweisend eine Mischung aus mikrofibrillärem Kollagen und superabsorbierenden Polymeren bekannt. Besagte Wundauflage soll aus einer Wunde austretendes Blut aufsaugen und das Gerinnen verursachen. Die verwendeten

### Materialien sind jedoch ziemlich teuer.

Weitere blutstillende Verbände werden in der US4616644 und der EP0206697A2 offenbart. Hier wird eine dünne Schicht von hochmolekularen Polyethylenoxids an der Oberfläche einer perforierten Plastikfolie verwendet. Solche Materialien interagieren jedoch nicht mit den natürlich vorkommenden blutstillenden Agenzien.

Aus der US6967261 ist ein Verband bekannt, der eine Schicht aufweisend ein quervernetztes Polyethylenoxid-Harz (superabsorbierend) sowie blutstillende Agenzien aufweist. Bei besagten Agenzien kann es sich z.B. um Chitosan-Niacinamid-Ascorbat, Chitosan oder Natriumalginat handeln.

Aufgabe der vorliegenden Erfindung ist es, einen Wundpflegeartikel bereitzustellen, der die Nachteile der aus dem Stand der Technik beschriebenen Artikel vermeidet.

Diese Aufgaben werden mit den Merkmalen des vorliegenden Anspruchssatzes gelöst. Die Unteransprüche geben bevorzugte Ausführungsformen an. Dabei ist zu beachten, dass die genannten Bereichsangaben durchweg einschließlich der jeweiligen Grenzwerte zu verstehen sind.

Demnach ist ein Wundpflegeartikel vorgesehen, aufweisend eine Menge an superabsorbierenden Polymeren. Der Wundpflegeartikel ist **dadurch gekennzeichnet, dass** er mindestens ein Agenz aufweist, das die Blutung oder die Blutungsneigung einschränken kann.

Bei den besagten superabsorbierenden Polymeren handelt es sich um Kunststoffe, die in der Lage sind, ein Vielfaches ihres Eigengewichts - bis zum 1000-fachen - an Flüssigkeit aufzunehmen. Diese Kunststoffe können In Form eines Granulats, eines Pulvers, einer Schüttung, eines Presslings, eines Schaums, in Form von Fasern, eines Fasergewirkes, - geleges oder -vlieses und/oder einer Faserwatte vorliegen.

Chemisch handelt es sich dabei in der Regel um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird oft ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk - das Wasser kann ohne Hilfe nicht mehr entweichen.

Superabsorbierende Polymere finden größtenteils in Babywindeln, jedoch auch in Produkten fiir die Damenhygiene, für die Inkontinenzversorgung oder die Behandlung von nicht blutenden, dafür chronisch exsudierenden Wunden statt.

Das Aufnahmevermögen für Flüssigkeiten ist stark von der Zusammensetzung der Flüssigkeit abhängig. Das beste Aufnahmevermögen existiert gegenüber destilliertem Wasser. Jedoch schon gegenüber Urin mit einem Salzgehalt von etwa 2 Gew.-% ist eine deutliche verminderte Aufnahmekapazität zu beobachten. Blut, das einen Feststoffanteil (Hämatokrit) von etwa 40 % und darüber hinaus einen hohen Anteil an gelösten Salzen im Serum aufweist, wird noch schlechter aufgenommen.

Bevorzugt ist dabei vorgesehen, dass es sich bei dem Agenz um mindestens einen chemisch und/oder pharmakologisch und/oder physiologisch wirkenden Wirkstoff bzw. Wirkstoffkomplex handelt. Ebenso kann es sich bei dem Agenz um mindestens ein physikalisch wirkendes Wirkelement handeln.

Die Begriffe "chemisch und/oder pharmakologisch und/oder physiologisch wirkender Wirkstoff bzw. Wirkstoffkomplex" und "physikalisch wirkendes Wirkelement" werden noch erläutert werden.

Bevorzugt handelt es sich bei dem chemisch und/oder pharmakologisch und/oder physiologisch wirkenden Wirkstoff bzw. Wirkstoff komplex um mindestens einen Stoff bzw. eine Zusammensetzung ausgewählt aus der Gruppe enthaltend:
- Tranexamsäure
- Blutstillende Watte, aufweisend oxidierte Zellulose und/oder Eisenchlorid
- Blutstillende Mittel aus der Zahnmedizin
- Gelatine, bevorzugt boviner, equiner und/oder porciner Genese, besonders bevorzugt rekombinanter Genese
- Pflanzliche Gelatine
- Präparate auf der Basis von Oligo- oder Polysacchariden, wie Stärke, Kartoffelmehl
- oder Kartoffelstärke, bevorzugt Oligo- oder Polysaccharide der Glucose
- Oxidierte regenerierte Zellulose
- Substituierte Cellulose, bevorzugt Carboxyalkylierte Cellulose, besonders bevorzugt Carboxymethylcellulose
- Fibrin verklebende Stoffe, ggf. humaner Genese
- Blutgerinnungsfaktoren I bis XII, insbes. VII bis IX (ggf. auch aktiviert)
- Hemmer der Inhibitoren der Blutgerinnungsfaktoren
- Plasmaprotein, human mit Faktor VIII-Inhibitor
- Kollagen, bevorzugt boviner, porciner und/oder equiner Genese, besonders bevorzugt
- rekombinanter Genese
- Pflanzliches Kollagen, beispielsweise aus Seegras oder Akazien oder Weizenproteinen
- Extensin, ein pflanzliches Glycoprotein mit hohem Hydroxyprolingehalt
- Thrombozytenaggregationsförderer
- Antagonisten der Hemmer der Cyclooxygenase 1 und 2
- Adenosindiphosphat
- Lipoxine
- Leukotriene
- Hemmer der Prostaglandine
- Derivate der Arachidonsäure
- Thromboxane wie TXA2 und TXB2, PGH2
- Epinephrin
- Fibrinspaltprodukte
- Antifibrinolytika
- Antagonisten zum Tissue Factor Pathway Inhibitor (TFPI),
- Antagonisten zu Thrombomodulin zusammen, mit Protein C und S,
- Antagonisten zu Antithrombin III
- Antagonisten zu Heparin bzw. Heparansulfaten
- Antagonisten zu a2-Makroglobulin
- Antagonisten zu C 1 -Inhibitoren
- Thrombin, Prothrombin und Prothrombinaktivator
- thrombozytäre Gerinnungsfaktoren
- Thrombomodulin
- Tretinoin
- Thrombin-aktivierter Fibrinolyseinhibitor (TAFI)
- chemisch definierte oder enzymatische Antihämorrhagika, bevorzugt Antifibrinolytika, z.B. auf der Basis von Aminosäuren wie Tranexamsäure, 4-(Aminomethyl)benzoesäure, Phytomenadion (Vit.K₁), Terlipressinacetat,
- Proteinase-Inhibitoren wie Aprotinin-Lösungen
- Vitamine der K-Cruppe
- gerinnungsfähiges Fibrinogen
- Eptacog alfa (aktiviert)
- Nonacog alfa
- humanes Thrombin
- Calciumionen-Donatoren, wie Calciumchrorid-Lösung
- Fibronektin
- Plasmafibronektin
- basisches Bismutgallat
- Tannin
- Metallische Plättchen oder Platten, die eine kühlende, komprimierende und/oder adstringierende Wirkung ausüben
- Adstringierend wirkende Mittel
- Alaunsalze, wie Kaliumaluminiumsulfat und Ammoniumaluminiumsulfat
- Sympathomimetika, wie Xylometazolin, Oxymetazolin, Katecholamine wie Adrenalin und Noradrenalin
- Methylxanthine wie Coffein und Teein
- Nikotin
- Chitosan
- Natriumalginat
- Chitosan-Niacinamid-Ascorbat
- Monsel's Lösung
- Eisensulfat

Weiterhin kann es sich bei dem chemisch und/oder pharmakologisch und/oder physiologisch wirkenden Wirkstoffbzw. Wirkstoffkomplex um mindestens einen Extrakt aus einer Heilpflanze handeln, ausgewählt aus der Gruppe enthaltend:
- Pflanzen der Gattungen Calendula, Echinacea, Pelargonium (bevorzugt *P. sidoides*)
- Ackerschachtelhalm (Equisetum arvense)
- Akanthus (Acanthus mollis)
- Akelei (Aquiiegia vulgaris)
- Arnika (Arnica montana)
- Bachehrenpreis (Veronica beccabunga)
- Barbarakraut (Barbarea vulgaris)
- Beinwell (Symphytum officinaie)
- Benediktenkraut (Cnicus benedictus)
- Besenrauke (Descurainia sophia)
- Betonie (Szachys officinalis)
- Bibernelle (Pimpinella major)
- Blutwurz (Potentilla erecta)
- Braunelle (Prunella vulgaris)
- Braunwurz (Scrophularia nodosa)
- Edelrauten (Artemisia)
- Efeu (Hedera helix)
- Fetthenne (Sedum telephium)
- Fingerkraut (Potentilla reptans)
- Gamander (Teucrium chamaedrys)
- Gänseblümchen (Bellis perennis)
- Gänsefingerkraut (Potentilla anserina)
- Gilbweiderich (Lysimachia vulgaris)
- Goldrute (Solidago virguarea)
- Gundermann (Glechoma hederaceum)
- Günsel (Ajuga reptans)
- Habichtskraut (Hieracium pilosella)
- Hängebirke (Betula pendula)
- Hauswurz (Sempervivum tectorum)
- Heidelbeere, hier: Saft (Vaccinium myrtiilus)
- Hirschzunge (Phyilitis scolopendrium)
- Hirtentäschelkraut (Capsella bursa-pastoris)
- Huflattich (Tussilago farfara)
- Hundrose, "Hagebutte" (Rosa canina)
- Kamille (Matricaria chamomilla)
- Katzenminze (Nepeta cataria)
- Katzenpfötchen (Antennaria dioica)
- Keulenbärlapp (Lycopodium clavatum)
- Klebkraut (Galium aparine)
- Klette (Arctium lappa)
- Knoblauchgamander (Teucrium scordium) zum Pulver (geruchslos)
- Knoblauchsrauke (Alliaria petiolata
- Königsfarn (Osmunda regalis)
- Kreuzkraut (Senecio vulgaris)
- Labkraut (Galium verum)
- Lavender (Lavendula officinalis)
- Löffelkraut (Cochlearia officinalis
- Myrte (Myrtus communis)
- Nabelkraut (Timbicilus pendulinus)
- Nelkenwurz (Geum urbanum)
- Odermennig (Hgrimonia eupatoria)
- Osterluzei (Aristolochia clematitis)
- Pestwurz (Petasites hybridus)
- Pfaffenhütchen (Euonymus europaea)
- Pfennigkraut (Lysimachia nummularia)
- Quendelseide (Cuscuta epithymum)
- Rainkohl (Lapsana communis)
- Rapunzelglockenblume (Campanula rapunculus)
- Roßkastanie (Aesculus hippocastanum)
- Ruprechtskraut (Geranium robertianum)
- Salbei (Salvia officinalis)
- Sanikel (Sanicula europaee)
- Schafgarbe (Achillea millefoiium)
- Schlangenknöterich (Polygonum bistorta)
- Schwarzerle (Alnus glutinosa)
- Schwarzpappel (Populus nigra)
- Silberdistel (Carlina acaulis)
- Stachelbeere (Ribes uva-crispa)
- Steinklee (Welilotus officinalis)
- Strandbeifuß (Artemisia maritima)
- Taubnessel (Lamium album)
- Tausendgüldenkraut (Centaurium erythraea)
- Teufelsabbiß (Succisa pratensis)
- Thymian (Thymus vulgaris)
- Tüpfeljohanniskraut (iiypericum perforatum)
- Vogelknöterich (Polygonum aviculare)
- Vogelmiere (Stellaria media)
- Wacholder (Juniperus comxunis)
- Waidehrenpreis (Veronica officinalis)
- Waldgeißblatt (Lonicera periclymenum)
- Waldmeister (Galium odoratum)
- Wasserdost (Eupatorium cannabinum)
- Wasserpfeffer (Polygonum hydropiper)
- Weidenröschen (Epilobium augustifolium)
- Wermut (Artemisia absinthium)
- Wiesenknopf, Groß (Sanguisorba officinalis)
- Wiesenknopf, Klein (Sanguisorba minor)
- Wundklee (Anthyllis vulneraria)
- Wurmfarn (Dryopteris filix-mas)
- Balsamkraut; Marienkraut (Tanaceum balsamica)
- Birnbaum (Pyrus communis)
- Eberraute (Artemisia abrotanum)
- Essigrose (Rosa gallica)
- Kohl (Brassica oleracea)
- Lauch (Allium porrum)
- Madonnenliiie (Lilium candidum)
- Walnußbaum (Juglans regia)
- Zwiebel (Allium cepa)
- Benzoebaum (Styrax tonkinensis)
- Kolabaum (Cola acuminata)

Der chemisch und/oder pharmakologisch und/oder physiologisch wirkende Wirkstoff bzw. Wirkstoffkomplex kann in trockener Form vorliegen und z.B. in ein Faservlies, das Bestandteil des erfindungsgemäßen Wundpflegeartikels ist, eingearbeitet sein. Er kann aber auch in gelöster Form vorliegen. Hierzu kann eine Lösung oder ein Gel hergestellt werden, die bzw. das besagten Wirkstoff bzw. Wirkstoffkomplex enthält. Bevorzugt kommt dabei ein wässriges und/oder alkoholisches Lösungsmittel zum Einsatz. Mit dieser Lösung bzw. diesem Gel kann z.B. eine Wundauflage, die Bestandteil des erfindungsgemäßen Wundpflegeartikels ist getränkt werde. Ein solcher Artikel kann vorkonfektioniert vorliegen oder auch in Form eines Kits vorliegen, bestehend beispielsweise aus einer Wundauflage und einem Gel enthaltend besagten chemisch und/oder pharmakologisch und/oder physiologisch wirkenden Wirkstoff bzw. Wirkstoffkomplex.

Grundsätzlich erscheint es zunächst einmal widersprüchlich, superabsorbierende Polymere in einem Wundpflegeartikel zu verwenden, der zur Blutungsstillung eingesetzt werden soll Ziel der Behandlung soll es in solchen Fällen ja gerade sein, das weitere Austreten von Flüssigkeiten, insbesondere von Blut, aus der Wunde zu verhindern, und nicht etwa, Flüssigkeiten austreten zu lassen und aufzunehmen.

Dies ist insbesondere deswegen von Bedeutung, weil Wundauflagen enthaltend Superabsorber mitunter sehr große Flüssigkeitsmengen aufnehmen können. So nimmt das Produkt Sorbion Sachet S 20-10 des Herstellers Sorbion Deutschland bis zu 400 ml an Flüssigkeit auf.

Der von den Erfindern verfolgte Ansatz, den erfindungsgemäßen Wundpflegeartikel zu verwenden, vermeidet daher einen unmittelbaren Kontakt zwischen einer akuten, stark blutenden Wunde und dem Wundpflegeartikel. Es sind dabei insbesondere zwei Verwendungsarten vorgesehen:

Bei chronischen Wunden, die ggf. eine leichte Blutung aufweisen, wie sie z.B. bei Ulcus cruris, Dekubitus oder ähnlichen Erkrankungen auftreten, ist vorgesehen, dass die Wundauflage in unmittelbaren Kontakt mit der Wunde gebracht wird. Hier ist die Gefahr eines kritischen Blutverlustes nicht zu befurchten. Es steht hier einerseits die Aufnahme von Exsudaten sowie die Einschränkung der Restblutung bzw. der Blutungsneigung durch dass bzw. die betreffenden Agenzien im Vordergrund. Anfänglich weiter austretendes Blut oder zumindest dessen seröse Anteile sollen hier überdies durch den Superabsorber der Wundauflage aufgenommen werden.

Gerade bei chronischen Wunden kann der Wundpflegeartikel jedoch auch als Kompressionsverband ausgebildet und/oder in ein solchen integrierbar sein. Dies ist insbesondere bei solchen Wunden angezeigt, die ödematösen Charakter haben, so z.B. Ulcus cruris. Hier verbessert die Kompression den Zustand der Gefäßwände, d.h. das endotheliale Zellgefuge wird rekonstruiert, und die interstitiellen Zwischenräume, durch welche Flüssigkeit in das Gewebe austreten kann, werden vermindert. Hinzu kommt, dass die Funktionalität der Venenklappen durch die Kompression wieder hergestellt wird und so der Venenstau reduziert wird. Eine Kombination einer solchen Kompression mit einem erfmdungsgemäßen absorbierenden Wundpflegeartikel, der ein chemisch und/oder pharmakologisch und/oder physiologisch wirkenden blutstillenden Wirkstoff bzw. Wirkstoffkomplex aufweist, ist sehr erfolgversprechend. Der Wundpflegeartikel nimmt dabei primär Exsudate auf, jedoch auch Blut, das anfänglich trotz des verabreichten blutstillenden Wirkstoffs bzw. Wirkstoffkomplexs aus der chronischen Wunde austritt.

Bei akut blutenden Wunden, wie sie z.B. durch ein Akuttrauma hervorgerufen werden, kann hingegen vorgesehen sein, dass
a) der Wundpflegeartikel als Druck- oder Kompressionsverband ausgebildet und/oder in ein solchen integrierbar ist, und/oder
b) der Wundpflegeartikel stromabwärts einer arteriellen Abbindung angeordnet ist.

In diesen Fällen liegt die Hauptlast der Blutstillung bei dem Druck- oder Kompressionsverband bzw. bei der Abbindung. Bei beiden handelt es sich also um physikalisch wirkende Wirkelemente im obigen Sinne. Dem Wundpflegeartikel obliegt dabei die Aufgabe, anfänglich weiter austretendes Blut durch den Superabsorber aufzunehmen.

Weitere physikalisch wirkende Wirkelemente im Sinne der Erfindung sind z.B. Wirkelemente, die Kälte in die Wunde einbringen, z.B. die sogenannten Kühlakkus, aber auch flüchtige Substanzen, die Verdunstungskälte erzeugen, so z.B. Ethanol, Isopropanol oder andere organische Lösungsmittel.

Im Zusammenhang mit dem oben gesagten kommt den verwendeten superabsorbierenden Polymeren eine besondere Bedeutung zu.

Da diese zwar Flüssigkeiten aufnehmen, nicht jedoch die zellulären Bestandteile des Blutes, kommt es an der Außenseite des erfindungsgemäßen Wundpflegeprodukts zu einer Agglomeration zellulärer Bestandteile.

Der Wundpflegeartikel kann bevorzugt einen im wesentlichen flachen Materialabschnitt aus Absorptionsmaterial aufweisen, der aus einem aufsaugenden Vlies mit darin verteilten superabsorbierenden Polymeren besteht. Diese können in Form von Partikeln, aber auch in Form von Fasern bzw. einem Faserverbund, in Form eines Schaumes oder dergleichen vorliegen. Ferner kann der Wundpflegeartikel eine flüssigkeitsdurchlässige Hülle aufweisen, die den Materialabschnitt umgibt und eine Barriere gegen feste Ausscheidungen bildet und die den Durchtritt von anderen ausgetretenen Substanzen zu dem innerhalb der Hülle angeordneten Materialabschnitt aus Absorptionsmaterial ermöglicht. Die Hülle ist bevorzugt wenigstens teilweise von einer Naht abgeschlossen

Besonders bevorzugt weist der Materialabschnitt in Draufsicht auf seine Flachseite eine Fläche (F1) auf, welche in seinem nicht benetzten Zustand um 1 % bis 90% kleiner als die Fläche (F2) der flachgelegten Hülle ist und in der Hülle frei beweglich oder fixiert ist, wobei die Hülle oder eine ihrer Schichten ganzflächig Poren aufweist, welche jeweils kleiner als die unbenetzten superabsorbierenden Polymere sind.

Besonders bevorzugt ist vorgesehen, dass die Hülle in Draufsicht auf ihre Flachseite einen umlaufenden, über die Naht hinausragenden Überstand aufweist und der Absorptionskörper frei von harten, scharfen Kanten und Ecken ist.

Die Poren oder Maschen der Hülle sind bevorzugt 0,01 mm bis 2,0 mm, vorzugsweise 0,20 mm bis 0,50 mm gross. Weiterhin kann bevorzugt vorgesehen sein, dass die Poren oder Maschen durch die Fäden- oder Faserabschnitte begrenzt sind, welche im Schnitt durch die Hülle etwa bogenförmig sind und mit ihren Bogen-Scheiteln nach aussen zeigen.

Die innere Lage des Absorptionskörpers weist bevorzugt ein flächenspezifisches Gewicht von 100 g/m² - 600 g/m² auf, wobei der darin verteilte Anteil der superabsorbierenden Polymere im Bereich von 25-100 Gew.-% liegt. Besonders bevorzugt liegt der Anteil der superabsorbierenden Polymere im Bereich von 15-100 Gew.-%.

Die Umhüllung ist bevorzugt aus gewebten oder vliesartig zusammengesetzten Kunstfasern, wie Polypropylen- oder Polyethylenfasern, gebildet. Sie besteht bevorzugt aus einem aus einem Gewebe oder Vlies, das ein flächenspezifisches Gewicht im Bereich von 10 - 1000 g/m² aufweist, besonders bevorzugt 10 - 100 g/m².

Die Verbindung zwischen der Umhüllung und einem innerhalb angeordneten Materialabschnitt ist bevorzugt durch Klebungen, Verschweissungen, Nähte, Steppnähte, Bonding Points, Prägungen oder durch thermomechanische Bindungen bewerkstelligt.

Bei besagten Bonding Points handelt es sich um Presspunkte, die mithilfe einer Presse auf den Wundpflegeartikel aufgebracht wurden, und die für eine thermische und/oder physikalische

Verbindung der verschiedenen Schichten des Wundpflegeartikels sorgen. Die Bonding Points liegen in der Regel in einem regelmäßigen Muster vor.

Dabei sind für die Umhüllung insbesondere hydrophobe Materialien wie Polyurethan, Polyethylen oder Teflon besonders bevorzugt, da sich - bei entsprechender Ausgestaltung - mit ihrer Hilde der Flüssigkeitsdurchtritt modulieren lässt. So ist z.B. einen verzögerter Flüssigkeitsdurchtritt bei Verwendung von superabsorbierenden Polymeren durchaus sinnvoll, um eine zu schnelle Drainage der Wunde zu verhindern. Überdies weisen die meisten hydrophoben Materialien eine glatte und inerte Oberfläche auf, wodurch Verklebungen mit der Wunde vermieden werden.

Diese Modulation des Flüssigkeitsdurchtritts kann durch spezielle Maßnahmen gewährleistet werden. Dies können z.B. in die Hülle bzw. Kaschierung eingebrachte Perforierungen oder Poren sein. Ebenso kann vorgesehen sein, dass den oben genannten Maßnahmen zur flächigen bzw. punktuellen Verbindung zwischen der Hülle und dem flachen Materialabschnitt die Doppelrolle zukommt, eine Flüssigkeitsdurchlässigkeit der Hülle bzw. Kaschierung zu bewirken.

Hierbei ist insbesondere an die oben genannten Bonding Points gedacht. Diese weisen Vertiefungen des Hüllmaterials auf, auf deren Grund (d.h. in ihrem den flachen Materialabschnitt kontaktierenden bzw. in denselben eindringenden Bereich) das Material gedehnt ist und daher Mikroperforierungen aufweist, die einen Flüssigkeitsdurchtritt ermöglichen. In dieser Ausgestaltung haben die Bonding Points also neben der Aufgabe, die verschiedenen Schichten des Wundpflegeartikels miteinander zu verbinden, auch die Aufgabe, den Flüssigkeitsdurch- bzw. Eintritt zu gewährleisten. Hinzu kommt, dass die Bonding Points eine die Oberfläche vergrößernde Wirkung aufweisen, was die Saugeffizienz weiter erhöht.

Ebenso ist an die oben genannten Steppnähte gedacht. Auch diese können einen Flüssigkeitsdurchtritt durch das hydrophobe Hüllenmaterial gewährleisten.

Wundauflagen der genannten Art sind beispielsweise in der WO03094813, der WO2007051599 und der WO0152780 der Anmelderin der vorliegenden Erfindung offenbart. Der Offenbarungsgehalt der genannten Schriften sei dem Offenbarungsgehalt dieser Schrift vollumfänglich beigefiigt.

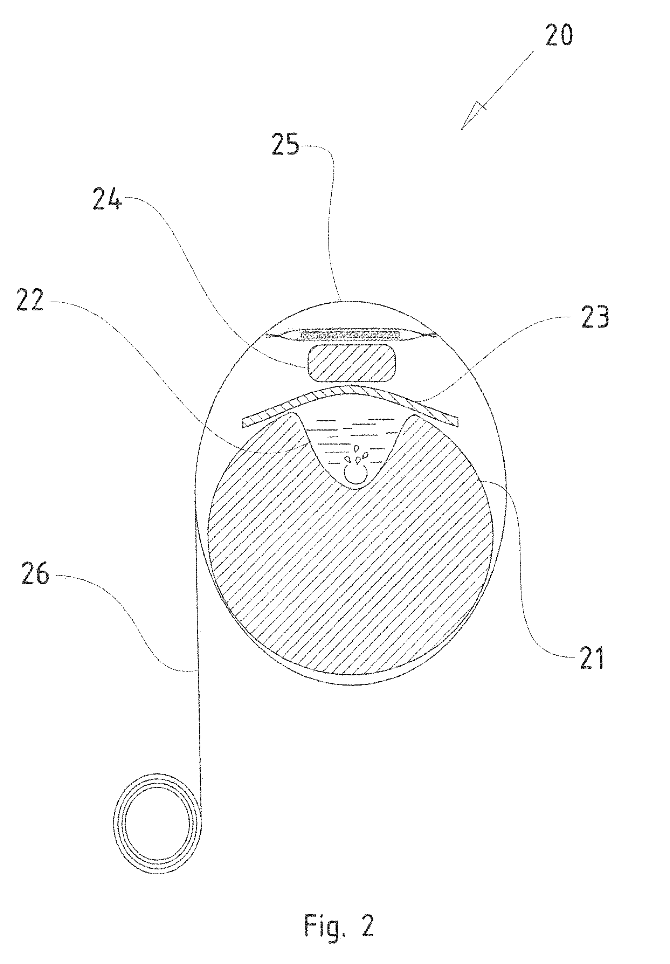

Die besagte Wundauflage enthält mindestens einen chemisch und/oder pharmakologisch und/oder physiologisch wirkenden Wirkstoff bzw. Wirkstoffkomplex und eignet sich insbesondere für die Blutstillung bei chronischen Wunden, die ggf. eine leichte Blutung aufweisen, oder bei akut blutenden Wunden als oder in Kombination mit einem Druck- oder Kompressionsverband (Fig. 2) bzw. stromabwärts einer arteriellen Abbindung.

Der besagte mindestens eine chemisch und/oder pharmakologisch und/oder physiologisch wirkende Wirkstoff bzw. Wirkstoff komplex ist dabei bevorzugt in dem der Wunde zugewandten Bereich der Wundauflage angeordnet.

In der Ausgestaltung mit einem Druck- oder Kompressionsverband ist überdies vorteilhaft, dass etwaige durchsickernde Flüssigkeit, die von den Superabsorbierenden Polymeren aufgenommen wird, zu einem Aufquellen letzterer fuhrt, wodurch wiederum der auf die Wunde ausgeübte physikalische Druck erhöht wird.

Weiterhin kann der Wundpflegeartikel eine primäre, nicht oder nur unwesentlich absorbierende Wundauflage aufweisen, die mindestens einen chemisch und/oder pharmakologisch und/oder physiologisch wirkenden Wirkstoff bzw. Wirkstoffkomplex aufweist, sowie eine peripher von dieser primären Wundauflage angeordnete sekundäre Wundauflage, die superabsorbierende Polymere enthält. Bei besagter sekundärer Wundauflage kann es sich z.B. um einen im wesentlichen flachen Materialabschnitt aus Absorptionsmaterial handeln, der aus einem aufsaugenden Vlies mit darin verteilten superabsorbierenden Polymeren besteht. Während die primäre Wundauflage die Aufgabe hat, die Blutung zu stillen, obliegt es der sekundären Wundauflage, weiterhin aus der Wunde austretendes Blut durch den Superabsorber aufzunehmen. Besagter Wundpflegeartikel kann weiterhin eine Trennschicht zwischen primärer und sekundärer Wundauflage aufweisen, die als Diffusionsbarriere fungiert und verhindert, dass der in der sekundären Wundauflage angeordnete Superabsorber eine Sogwirkung auf die Wunde ausübt und so die Blutung forciert. Besagte Diffusionsbarriere kann so ausgestaltet sein, dass Blut bzw. Serumflüssigkeit erst ab einem bestimmten hydrostatischen Druck durch die Barriere hindurchsickert und dann von der sekundären Wundauflage aufgenommen wird. Ebenso kann die Diffusionsbarriere Poren aufweisen, deren Durchmesser geringer ist als der der Blutzellen. So weisen Erythrocyten z.B. Durchmesser zwischen 6 und 10 µm auf, während Thrombozyten Durchmesser zwischen 1,5 und 3,0 µm aufweisen. Die Poren in besagter Diffusionsbarriere könnten daher z.B. Durchmesser zwischen 1 und 8 µm aufweisen.

U.U. ist eine solche Porengröße jedoch gar nicht erforderlich. Erythrozyten neigen unter dem Einfluß von Blutgerinnungsfaktoren und Thrombozyten zu einer Aggregation. Es ist daher damit zu rechnen, dass die Erythrozyten im Bereich der Wundauflage Aggregationen ausbilden, die wesentlich größere Durchmesser aufweisen als die Erythrozyten selbst, und auf diese Weise auch eine Diffusionsbarriere mit wesentlich höheren Porengrößen den Durchtritt von zellulären Bestandteilen wirksam verhindern kann.

Hinzu kommt, dass die Wundauflage bevorzugt Flüssigkeit aufnimmt und so zu einer Flüssigkeitsverarmung im Wundbereich führt. Dies erhöht wiederum die Aggregationsneigung der Erythrozyten, weil sich so der Hämatokrit im Milieu erhöht.

Die besagte Diffusionsbarriere kann dabei z.B. in Form einer Hülle ausgebildet sein, die um die sekundäre Wundauflage herum angeordnet ist. Diese Ausgestaltung entspricht beispielsweise in einer Ausgestaltung dem bereits beschriebenen Produkt Sorbion sachet.

Auch hier kann die Hülle Poren besagter Größe aufweisen, so dass die sekundäre Wundauflage lediglich die flüssigen Bestandteile des Blutes aufnimmt, nicht jedoch die zellulären Bestandteile, die vor der Hülle aufkonzentriert werden.

Ebenso kann die Diffusionsbarriere stark hydrophobe Eigenschaften aufweisen oder so ausgestaltet sein, dass ein Flüssigkeitskontinuum zwischen der Wunde und dem Superabsorber z.B. durch Lufteinschlüsse unterbunden ist. Die besagten Merkmale können auch untereinander kombiniert sein.

Hierzu kann die Diffusionsbarriere z.B. als eine semipermeable Folie bzw. Membran aus dem hydrophoben Material PTFE (Polytetrafluorethylen) ausgestaltet sein. Ebenso kann die Diffusionsbarriere als eine Schaummatte aus einem hydrophoben, Lufteinschlüsse aufweisenden Schaum ausgestaltet sein.

Weiterhin kann es sich bei der Diffusionsbarriere um eine ggf. mehrfach gelegte, dreidimensional perforierte Folie handeln, wie sie z.B. aus der DE 102006017194 der Anmelderin der vorliegenden Erfindung bekannt ist. Auch hier wird ein Flüssigkeitskontinuum zwischen der Wunde und dem Superabsorber z.B. durch Lufteinschlüsse unterbunden.

Weiterhin kann die besagte Diffusionsbarriere mindestens eine Lage aus oxidierter Zellulose aufweisen. Diese weist einerseits blutstillende Eigenschaften auf, andererseits kann die Porengröße so gewählt sein, dass Blutzellen diese Lage nicht passieren können.

Weiterhin kann es sich bei besagter Diffusionsbarriere um eine ggf. mehrfach gelegte, dreidimensional perforierte Folie handeln, wie sie z.B. aus der DE 102006017194 der Anmelderin der vorliegenden Erfindung bekannt ist, deren Offenbarungsgehalt vollumfanglich hier mit eingezogen werden soll. Auch hier wird ein Flüssigkeitskontinuum zwischen der Wunde und dem Superabsorber z.B. durch Lufteinschlüsse unterbunden.

Auch in dieser Ausgestaltung ist vorteilhaft, dass etwaige durchsickernde Flüssigkeit, die von den superabsorbierenden Polymeren aufgenommen wird, zu einem Aufquellen letzterer fuhrt, wodurch wiederum der auf die Wunde ausgeübte physikalische Druck erhöht wird.

Überdies kann ein solcher Wundpflegeartikel zusätzlich ein Druckpolster aufweisen. Bei dem Druckpolster kann es sich z.B. um ein flüssigkeitsdicht verpacktes Kissen aus Zellstoff handeln, ebenso aber z.B. um einen Knebel oder einen kugelförmigen Gegenstand.

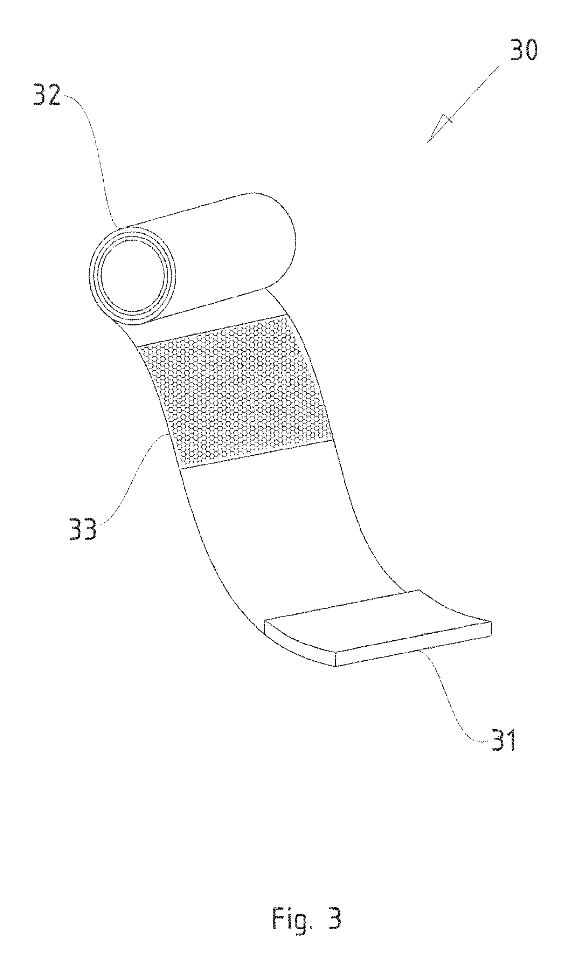

Auch kann der Wundpflegeartikel in Form eines Verbandpäckchens ausgestaltet sein, aufweisend eine primäre Wundauflage mit mindestens einem chemisch und/oder pharmakologisch und/oder physiologisch wirkenden Wirkstoff bzw. Wirkstoffkomplex sowie einem an der Wundauflage angeordneten Wickelabschnitt, der zumindest abschnittsweise superabsorbierende Polymere aufweist (Fig. 3). Überdies kann ein solcher Wundpflegeartikel zusätzlich ein Druckpolster aufweisen. Bei dem Druckpolster kann es sich z.B. um ein flüssigkeitsdicht verpacktes Kissen aus Zellstoff handeln, ebenso aber z.B. um einen Knebel oder einen kugelförmigen Gegenstand.

Auch in dieser Ausgestaltung ist vorteilhaft, dass etwaige durchsickernde Flüssigkeit, die von den superabsorbierenden Polymeren aufgenommen wird, zu einem Aufquellen letzterer führt, wodurch wiederum der auf die Wunde ausgeübte physikalische Druck erhöht wird.

Weiterhin kann der Wundpflegeartikel in Form eines Materialabschnitts mit einer Längserstreckung aufweisend Absorptionsmaterial ausgebildet sein, wobei der Materialabschnitt ggf. elastisch verformbare Eigenschaften aufweist, und wobei der Materialabschnitt superabsorbierende Polymere sowie ggf. mindestens einen chemisch und/oder pharmakologisch und/oder physiologisch wirkenden Wirkstoff bzw. Wirkstoffkomplex aufweist.

Dieser Materialabschnitt kann z.B. in Form eines Schlauchs um eine Gliedmaße angeordnet werden, die eine stark blutende Wunde aufweist. Aufgrund der elastischen Eigenschaften kann dabei ein Druck auf die Wunde aufgebracht werden, der zu einer physikalischen Blutungsstillung führt. Überdies können ggf. vorhandene chemisch und/oder pharmakologisch und/oder physiologisch wirkenden Wirkstoffe bzw. Wirkstoffkomplexe zu der Blutungsstillung beitragen. Etwaiges weiter austretendes Blut wird dann von den superabsorbierenden Polymeren aufgenommen.

Ein solcher Wundpflegeartikel kann auch als Abreißartikel ausgebildet sein. Hierzu ist das Material z.B. auf einer Spule oder Rolle angeordnet, und kann bei Bedarf in der entsprechenden Länge abgerissen werden. Hierzu kann das Material ggf. Querperforationen aufweisen, um das Abreißen zu erleichtern.

Ein solcher Artikel eignet sich besonders gut zur Wundversorgung in der Akut- und Notfallmedizin, insbesondere bei Verletzungen aufgrund von Unfällen, wie Arbeits- und Verkehrsunfällen, oder bei Schuß- oder Explosionsverletzungen. So kann ein solcher Artikel als Abreißartikel wie oben beschrieben ausgebildet Bestandteil einer Akut-, Notfall- oder Militärmedizinischen Ausrüstung sein, so z.B. eines Erste-Hilfe-Koffers oder -Päckchens, eines Notarztkoffers, eine Rettungs- oder Krankenwagenausstattung oder dergleichen.

Bevorzugt ist in diesem Zusammenhang außerdem vorgesehen, dass der Wundpflegeartikel außerdem mindestens einen nutritiven, mindestens einen desinfizierenden bzw. dekontaminierenden und/oder mindestens einen Proteasen hemmend wirkenden Wirkstoff und/oder Wirkstoffkomplex aufweist.

Bei dem desinfizierend wirkenden Wirkstoff und/oder Wirkstoffkomplex kann es sich z.B. um eine Zusammensetzung aus mindestens einem Vitamin oder Vitaminderivat, einem Metallion sowie einem Detergenz handeln. Ebenso kann es sich dabei um einen BLIS (bacteriocin like inhibitory substance) oder um beschichtete magnetische Partikel handeln.

Bei dem nutritiv wirkenden Wirkstoff und/oder Wirkstoff komplex kann es sich um eine Zusammensetzung enthaltend mindestens die Bestandteile eines enteralen und/oder parenteralen Diätetikums handeln. Ebenso kann es sich dabei um mindestens ein Wirkelement ausgewählt aus der Gruppe enthaltend Insulin, rekombinantes Insulin, Proinsulin, einen insulinähnlichen Wachstumsfaktor (Insulin-like growth factor, IGF), ein Insulinmimetikum und/oder einen diabetikerspezifischen, nicht glucose- bzw. saccharosebasierenden Energieträger handeln.

Bei dem Proteasen hemmend wirkenden Wirkstoff und/oder Wirkstoff komplex kann es sich um mindestens ein Wirkelement ausgewählt aus der Gruppe enthaltend Proteasehemmer, superabsorbierende Polymere, Chelatoren für zweiwertige Kationen, Kollagen, beschichtete magnetische Partikel, Säuren, Puffer, nicht pathogene säureproduzierende Mikroorganismen, Probiotika und/oder Symbiotika handeln.

Weitere Zusammenhänge und Hintergründe zu den nutritiven, einen desinfizierenden bzw. dekontaminierenden und/oder Proteasen hemmend wirkenden Wirkstoffen und/oder Wirkstoff komplexen sind in der DE 10200703 0931 der Anmelderin der vorliegenden Anmeldung beschrieben, auf deren Inhalt hier vollumfänglich Bezug genommen wird. In der DE 10200703 0931 sind auch weitere nutritive, desinfizierende bzw. dekontaminierende und/oder Proteasen hemmend wirkende Wirkstoffen und/oder Wirkstoffkomplexe beschrieben, die ebenfalls als in dieser Anmeldung offenbart gelten sollen.

Weiterhin kann der erfindungsgemäße Wundpflegeartikel auch in ein Wundversorgungssystem zur Wunddrainage unter Einsatz von Unterdruck eingebracht sein. Solche Systeme sind z.B. in den Schriften DE202004017052, WO2006048246 und DE202004018245 der Anmelderin der vorliegenden Erfindung offenbart, deren Offenbarungsgehalt der vorliegenden Erfindung als zugehörig betrachtet sein soll.

Aus erstgenannter ist eine Vorrichtung zur Wundbehandlung unter Einsatz von Unterdruck bekannt, aufweisend ein gasdichtes Wundabdeckungselement, das im am Körper des Patienten angelegten Zustand einen zwischen der jeweiligen Wunde und dem Wundabdeckungselement verbleibenden Raum bildet, und wenigstens eine Anschlussstelle, die mit dem Raum in Kontakt steht und über welche die im Raum befindliche Luft evakuiert werden kann, wobei das Wundabdeckungselement von wenigstens einem flächenhaften, die Wundsekrete aufnehmenden Wundpflegeartikel unterlegt ist, dessen Volumen im Laufe des Absorptionsprozesses zunimmt, so dass die absorbierten Wundsekrete innerhalb des Wundpflegeartikels und damit unterhalb des Wundabdeckungselementes bis zur Entfernung des Wundpflegeartikels aus dem Körper des Patienten verbleiben, der Wundpflegeartikel wenigstens eine Lage eines mit Superabsorbentien angereicherten Textilabschnittes ist, die mit einer flüssigkeitsdurchlässigen Hülle umgeben ist, und die Lage in Draufsicht auf ihre Flachseite eine Fläche hat, die 3% bis 90% kleiner als die der Hülle ist, damit sich der Wundpflegeartikel in der Nähe seiner gesamten Füllungskapazität im Querschnitt einer Kreisform annähern kann.

Aus zweitgenannter ist ein Mehrkomponentenverband zur Wundbehandlung des menschlichen oder tierischen Körpers unter Einsatz von Unterdruck bekannt, aufweisend:
ein Wundabdeckungselement zur Anbringung an Haut- und Schleimhautoberflächen, wenigstens eine Anschlussstelle., die mit dem Wundraum in Kontakt steht und über welche die im Wundraum befindlichen Stoffe evakuiert werden können, wobei dieser superabsorbierende Polymere aufweist, wobei die absorbierten Wundsekrete an Polymere gebunden im Wundraum bis zur Entfernung aus dem Wundraum verbleiben, wobei die Polymere durch ihre Bindungskapazität wechselseitige Synergien mit den subatmosphärischen Drücken unterstützen.

Aus letztgenannter ist eine Drainagevorrichtung zur Wundbehandlung unter Einsatz von Unterdruck bekannt, aufweisend ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Raum bildet, wenigstens einen Drainageschlauch, der in den Raum einsetzbar ist, über den die im Raum befindlichen Stoffe evakuiert werden können, und wenigstens einen innerhalb des Raumes angeordneten, die Wundsekrete aufsaugenden Wundpflegeartikel, der wenigstens eine Lage eines mit Superabsorbentien angereicherten Textilabschnittes aufweist, die mit einer flüssigkeitsdurchlässigen Hülle umgeben ist, wobei die absorbierten Wundsekrete innerhalb des Wundpflegeartikel und damit unterhalb des Wundabdeckungselementes bis zur Entfernung des Wundpflegeartikel aus dem Körper des Patienten verbleiben, und wobei das Wundabdeckungselement eine gasdicht verschliessbare Behandlungsöffnung aufweist, durch die der Wundpflegeartikel in den Raum einlegbar und aus dem Raum entnehmbar ist.

Der erfindungsgemäße Wundpflegartikel kann überdies einen an anatomische Gegebenheiten angepasste Form aufweisen. Hierzu kann er z.B. in Form einer Manschette ausgebildet sein; die über den einen Arm oder ein Bein oder ein Gelenk gestülpt werden kann, oder in Form eines an die Ferse, das Ellenbogengelenk oder dergleichen angepaßten Verbandes.

Der erfindungsgemäße Wundpflegartikel kann außerdem so ausgebildet sein, dass er sich zur Umlage um eine chirurgisch angelegte Leitung eignet. Hierzu kann der Wundpflegeartikel z.B. wenigstens einen Schlitz aufweisen, der es ermöglicht, den Verband am Körper eines Patienten um eine Leitung (z.B. eine Drainageleitung oder einen Kathether) umzulegen, wobei dem Wundpflegeartikel ein zweiter, ebenfalls flächenhafter Wundpflegeartikel zugeordnet ist, der von dem ersten Wundpflegeartikel in einem Abstand liegt, wobei der Abstand durch einen Verbindungsstreifen oder -steg überbrückt ist. Ein solcher Wundpflegartikel ist z.B. aus der DE202006005966 der Anmelderin der vorliegenden Erfindung bekannt, deren Inhalt vollumfänglich dem Offenbarungsgehalt der vorliegenden Beschreibung hinzugefügt werden soll.

Erfindungsgemäß ist überdies die Verwendung eines wie oben beschriebenen Wundpflegeartikels für traumatisch erzeugte Wunden vorgesehen. Hier ist insbesondere an Wunden gedacht, wie sie im Straßenverkehr, beim Sport, bei körperlicher Arbeit, bei Unfällen, durch Schusswaffen oder aufgrund von Verletzungsdelikten auftreten. All diese Wunden zeichnen sich in der Regel durch starke Blutungen aus, die häufig durch verletzte Arterien hervorgerufen werden. Grundsätzlich kommen dabei besonders bevorzugt die erwähnten physikalisch wirkenden Wirkelemente in Frage.

Ebenso ist erfindungsgemäß die Verwendung eines wie oben beschriebenen Wundpflegeartikels für militärische Zwecke vorgesehen. Hier steht insbesondere die Versorgung von Feldverletzungen im Vordergrund, wie sie z.B. durch Schusswaffen, Sprengkörper oder dergleichen hervorgerufen werden. Auch hier kommen besonders bevorzugt die erwähnten physikalisch wirkenden Wirkelemente in Frage.

In diesem Zusammenhang sind auch die desinfizierenden und dekontaminierenden Eigenschaften der Superabsorbierenden Polymere von Bedeutung. So können superabsorbierende Polymere aufgrund ihrer hohen Aufnahmekapazität sowohl

Krankheitserreger als auch Giftstoffe und strahlende Partikel aufnehmen und immobilisieren. Aus diesem Grunde eignet sich der erfindungsgemäße Wundpflegeartikel auch für die Behandlung von Verletzungen, die durch Verstrahlungen, Verbrennungen, Verätzungen, Vergiftungen oder den Einfluß von biologischen Waffen. Der Wundpflegeartikel kann daher z.B. in der Erstversorgung atomar, biologisch oder chemisch kontaminierter Soldaten verwendet werden. Er muss nach Verwendung fachgerecht entsorgt werden.

Weiterhin ist erfindungsgemäß die Verwendung eines wie oben beschriebenen Wundpflegeartikels zur Behandlung von Wunden, die aufgrund einer Hämophilie-Erkrankung oder aufgrund eines systemisch bedingte oder pharmakologisch hervorgerufenen Gerinnungsdefizits einen verschlechterten Heilungsverlauf aufweisen, vorgesehen.

Hämophilie ist eine Erbkrankheit, bei der die Blutgerinnung gestört ist. Das Blut aus Wunden gerinnt nicht oder nur langsam. Häufig kommt es auch zu spontanen Blutungen, die ohne sichtbare Wunden auftreten. Hämophilie tritt hauptsächlich bei Männern auf, da sie in der Regel durch ein X-chromosomal-rezessives Gen bedingt ist

Ein pathologisches Gerinnungsdefizit kann z.B. durch eine Lebererkrankung, wie z.B. eine Leberzirrhose hervorgerufen werden.

Ein systemisch bedingtes Gerinnungsdefizit kann z.B. ebenfalls erblich bedingt sein, so z.B. die sogenannte APC-Resistenz, aber auch durch eine generell schlechte Gesamtkonstitution des Patienten bedingt sein. Hierzu kann auch die Fallkonstellation zählen, bei welcher der Patient zuvor eine Blut- oder Thrombocytenspende vorgenommen hat oder Gerinnungsfaktoren gespendet hat.

Ein pharmakologisch hervorgerufenes Gerinnungsdefizit kann z.B. im Rahmen einer Herzinfarkt-, einer Schlaganfall- oder eine Thrombolyse-Therapie bzw. Prophylaxe auftreten. Ursache hierfür sind dem Patienten verabreichte Gerinnungshemmer, wie z.B. Marcumar, oder sogenannte Blutverdünner, wie z.B. Acetylsalicylsäure. Ebenso treten pharmakologisch hervorgerufene Gerinnungsdefizite nach Chemotherapien und/oder Bestrahlungen im Rahmen einer Krebsbehandlung auf.

Für all diese Indikationen kann die Verwendung des erfindungsgemäßen Wundpflegeartikels von großem Vorteil sein. Grundsätzlich kommen dabei besonders bevorzugt die erwähnten chemisch und/oder pharmakologisch und/oder physiologisch wirkenden Wirkstoffe bzw. Wirkstoffkomplexe in Frage.

Weiterhin ist erfindungsgemäß die Verwendung eines wie oben beschriebenen Wundpflegeartikels zur operativen bzw. postoperativen Versorgung vorgesehen. Auch bei solchen Indikationen ist eine schnelle Blutstillung häufig oberstes Gebot. Grundsätzlich kommen hier sowohl die erwähnten chemisch und/oder pharmakologisch und/oder physiologisch wirkenden Wirkstoffe bzw. Wirkstoffkomplexe als auch die erwähnten physikalisch wirkenden Wirkelemente in Frage.

### Definitionen

Der Begriff' Wundpflegeartikel" soll im Folgenden insbesondere eine Wundauflage, bevorzugt eine flächige Wundauflage oder ein Wundpflegetuch bezeichnen. Besagte

Wundauflage kann dabei sowohl absorbierend als auch nicht oder nur unwesentlich absorbierend ausgestaltet sein. Insbesondere kann der Begriff "Wundpflegeartikel" auch als ein Ensemble verschiedener Produkte verstanden werden, die in einer gegebenen Anordnung auf der zu behandelnden Wunde angeordnet werden. Dieses Ensemble kann eine physikalische Einheit bilden, indem die verschiedenen Produkte in einer gemeinsamen Hülle zusammengefasst oder - ggf. ohne Hülle - adhäsiv miteinander verbunden sind. Das Ensemble kann jedoch auch in Form eines Kits vorliegen, bei dem die verschiedenen Produkte mithilfe einer Wickel in der gegebenen Anordnung auf der zu behandelnden Wunde angeordnet sind.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefordert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Unter dem Begriff "chemisch und/oder pharmakologisch und/oder physiologisch wirkender Wirkstoff bzw. Wirkstoffkomplex" sollen im Folgenden solche Wirkstoffe bzw. Wirkstoffkomplexe verstanden werden, die die Blutung oder die Blutungsneigung einzuschränken imstande sind. Der Wirkungsweg ist hier eine chemische, pharmakologische und/oder physiologische Interaktion mit dem Wundmilieu.

Unter dem Begriff "Wirkstoffkomplex" soll im Folgenden nicht nur ein Komplex im chemischen Sinne verstanden werden, sondern insbesondere eine Zusammensetzung synergistisch eine Wirkung hervorrufender Wirkstoffe.

Unter dem Begriff "physikalisch wirkendes Wirkelement" soll im Folgenden ein Wirkelement verstanden werden, das auf physikalischem Wege, d.h. durch die Ausübung von Druck, Zug, Kälte und dergleichen, die Blutung oder die Blutungsneigung einzuschränken imstande ist. Ebenso kann ein solches Wirkelement durch inhärente physikalische Eigenschaften oder durch physikalische Interaktionen eines Wirkstoffs oder Wirkstoffkomplexes, wie z.B. die Ausübung von Kapillarkräften auf das Milieu, wirken.

Unter dem Begriff "Abbindung" soll im Folgenden eine notfallmedizinische Maßnahme verstanden werden, die in der Lage ist, den arteriellen Blutfluß z.B. in einer Gliedmaße zu stoppen, um so einen unvertretbaren Blutverlust in einer Wunde zu verhindern. Indikation für eine solche Abbindung sind in der Regel traumatische Einwirkungen, die zu einer Verletzungen mindestens einer Arterie fuhren.

Unter dem Begriff "stromabwärts einer Arteriellen Abbindung" soll im Folgenden eine Position am Körper eines menschlichen oder tierischen Patienten verstanden werden, die sich in Bezug auf den arteriellen Blutfluß vom Herz aus gesehen distal einer arteriellen Abbindung befindet.

Unter dem Begriff "chronische Wunden" sollen Wunden verstanden werden, die nicht primär auf traumatische Einwirkungen zurückgehen. Zwar können traumatische Einwirkungen der ursprüngliche Auslöser einer solchen Wunde gewesen sein, aber die chronische Wunde zeichnet sich vor allem durch eine verzögerte Wundheilung auf. Chronische Wunden weisen häufig - wenn überhaupt - nur leichte Blutungen auf, dafür oftmals eine starke Exsudation.

Unter dem Begriff "leichte Blutung" soll eine Blutung verstanden werden, die nicht arteriellen Ursprungs ist, sondern ggf. venösen Ursprungs oder interstitiellen bzw. kapillaren Ursprungs, und die in jedem Fall so leicht ausfällt, dass sie nicht mittel- oder unmittelbar lebensbedrohend ist.

Unter dem Begriff "akut blutende Wunden" sollen solche Wunden verstanden werden, die zu großen Blutverlusten führen. In der Regel sind hierfür arterielle Blutungen verantwortlich, die z.B. durch traumatische Einwirkungen verursacht werden. Akut blutende Wunden können u.U. mittel- oder unmittelbar lebensbedrohend sein. Aus diesem Grunde hat bei akut blutenden Wunden die Blutungsstillung eine sehr hohe Priorität.

Unter dem Begriff "Druckverband" soll im Folgenden der aus der Notfallmedizin bekannte Druckverband verstanden werden, Dieser besteht aus einem nicht zu harten, nicht saugfähigen Gegenstand (Druckpolster) ohne scharfe oder harte Kanten, der auf eine bereits abgedeckte Wunde aufgebracht wird und mithilfe einer Wickel mit mäßigem Zug befestigt wird. Durch den ausgeübten Druck wird die Durchblutung des betreffenden Körperteils gemindert und die traumatisch geöffneten Blutgefäße werden wieder geschlossen. Ein solcher Druckverband ist symbolhaft in Fig. 2 dargestellt.

Unter dem Begriff "nicht oder nur unwesentlich absorbierende Wundauflage" soll eine Wundauflage bezeichnen, die ein geringes Absorptionsvermögen für Flüssigkeiten aufweist. Insgesamt soll das Absorptionsvermögen dabei bei weniger als 60 Gew.-%, bevorzugt weniger als 20 Gew.-% des Trockengewichts der Wundauflage liegen. Primäre Aufgabe einer solchen Wundauflage ist daher auch nicht die Aufnahme von Blut oder Exsudaten, sondern die Abgabe blutstillender Agenzien im Sinne der vorliegenden Erfindung.

Unter dem Begriff "Kompressionsverband" wird hingegen in aller Regel ein Verband verstanden, der ähnlich wie ein Druckverband wirkt, jedoch auf das genannte Druckpolster verzichtet. Der Druck oder die Kompression auf die Wunde wird hierbei ausschließlich durch die Wickel ausgeübt. Hierbei kann das Wickelmaterial elastisch sein.

Gemäß der vorliegenden Erfindung kann der erfindungsgemäße Wundpflegeartikel in einen solchen Druck- oder Kompressionsverband integriert oder selbst als Druck- oder Kompressionsverband ausgebildet sein.

### Zeichnungen und Beispiele

Die vorliegende Erfindung wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken.

### Beispiel 1

Substituierte Cellulose basiert auf Cellulose, die chemisch modifiziert wurde. Bei Cellulose handelt es sich um ein unverzweigtes Polymer aus 1-4-β-glykosidisch verknüpften Glucosemolekülen mit einer Kettenlänge zwischen einhundert und zehntausend Monomeren.

Bevorzugt handelt es sich bei der Substituierten Cellulose um Celluloseether, wie z.B. alkylierte Cellulose (z.B. Methylcellulose, Ethylcellulose, Propylcellulose), hydroxialkylierte Cellulose (z.B. Hydroximethyl-Cellulose, Hydroxiethyl-Cellulose, Hydroximethyl-Cellulose, Hydroxipropyl-methyl-Cellulose) oder carboxyalkylierte Cellulose (z.B. Carboxymethylcellulose, Carboxyethylcellulose, Carboxypropylcellulose).

Bevorzugt ist vorgesehen, dass die modifizierte Cellulose einen Substitutionsgrad zwischen einschließlich 0,05 und einschließlich 3 Alkyl-, Hydroxialkyl- oder Carboxyalkylgruppen pro Glucoseeinheit aufweist. Dabei bezeichnet ein Substitutionsgrad von 3 drei Substituenten pro Glucoseeinheit, während ein Substitutionsgrad von 0,05 einen Substituenten pro 2000 Glucoseeinheiten bezeichnet.

Besagte chemisch modifizierte Cellulosen gehen bei Kontakt mit Wasser sukzessive in einen gelartigen Zustand über. Dabei ist die gelbildende Eigenschaft abhängig vom Substitutionsgrad der Cellulose, d.h. je höher der Substitutionsgrad, desto früher tritt die Gelbildung ein.

Substituierte Cellulose, insbesondere CMC, hat eine Blutstillende Wirkung, die einerseits auf mechanische und andererseits auf blutphysiologische Ursachen zurückgeht.

So beschleunigt CMC die Agglutination der Blutplättchen (Thrombocyten) über Fibrinogen- oder Fibrinmonomere und beschleunigt auch die Akkumulation der Fibrinmonomere zu Fibrinpolymeren. Dieses bedeutet, dass CMC physikalisch auf ein Fibrinogenmolekül oder Fibrinmonomer einwirkt. Darüber hinaus hat CMC eine fördernde Wirkung auf die Zelladhäsion und fördert auch so die Blutstillung.

Durch die Eigenschaft von CMC, bei Kontakt mit Feuchtigkeit ein Gel auszubilden, wird überdies die Blutung auch mechanisch gestoppt.

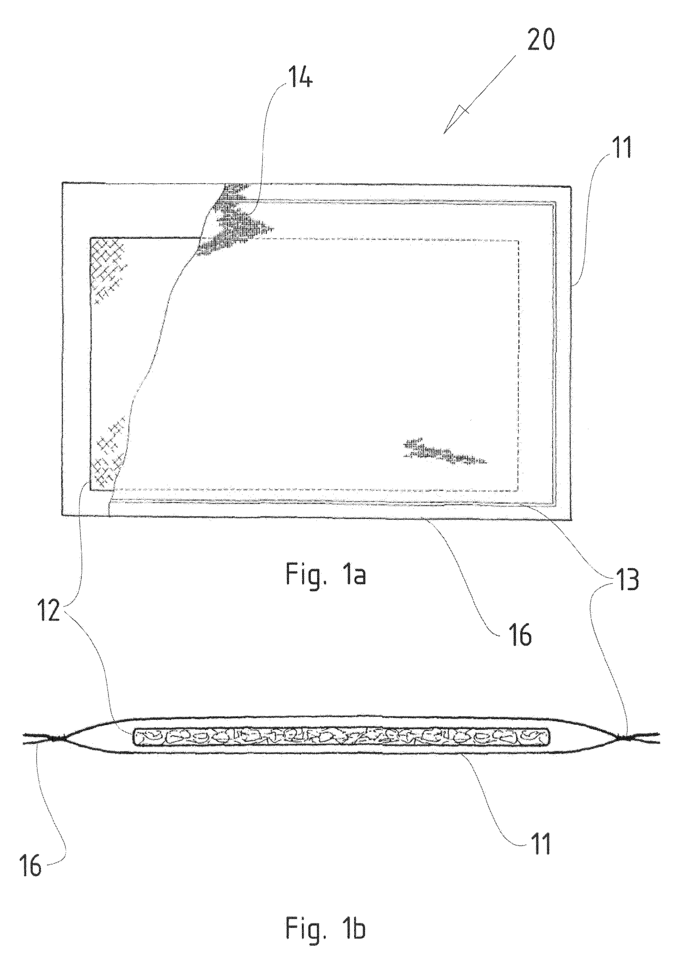

Figs. 1a, 1b zeigt einen Absorptionskörper 10, der aus einer Hülle 11 und einem darin lose untergebrachten Absorptionsmaterials in Form eines flachen, zelluloseartigen Materialabschnittes 12 besteht.

Der Materialabschnitt weist eine Dicke etwa 2 mm auf und ist weist ein superabsorbierendes Polymer auf. Das Polymer kann in Pulver- oder Granulatform, in Form von Fasern, einer Watte, einem Vlies oder einem Schaum vorliegen. Das besagte Pulver kann dabei als Schüttung, als Preßling oder eingebettet in ein Faservlies ("Airlaid") vorliegen. Die Hülle 11 gemäss Fig. 1 ist aus zwei gleichen, an ihrer Peripherie miteinander über eine Schweissnaht 13 verbundenen Seitenwänden gefertigt. Die Fläche F1 des Materialabschnitts 12 macht dabei etwa 2/3 der Fläche F2 der Hülle 11 aus. Durch die Unterschiede zwischen den Flächen F1.und F2 der Hülle 11 und des Absorptionsmaterials 12, kann sich das Aufsaugvermögen des Absorptionsmaterials voll entfalten. Die Hülle 11 weist eine kleinmaschige Struktur auf, deren Poren 14 im vorliegenden Fall etwa 0,15 mm bis 0,25 mm gross sind, und kann beispielsweise aus modifizierter Cellulose (bevorzugt CMC) bestehen.

Eine Besonderheit des Absorptionskörpers 10 ist ein umlaufender, über eine Naht 13 hinausragender Überstand 16, dessen Breite zwischen 1 mm und 5 mm liegt. Die Aufgabe des Überstands 16 ist, eine sanfte Pufferzone zwischen dem Gewebe des Patienten und der Naht 13 zu bilden, wenn mit dem Absorptionskörper 10 unvorsichtig manipuliert wird, d. h. wenn der Absorptionskörper mit seiner Kante bzw. Ecke die schmerzhafte Wunde berührt.

Die Naht 13 ist bevorzugt klebstoff und/oder bindemittelfrei ausgeführt, z.B. als Schweißnaht, bevorzugt als Ultraschall-Schweißnaht.

Es ist vorgesehen, auch andere, insbesondere quadratische Hüllen mit Überstand 5, zu verwenden, bei denen die Fläche F1.des inneren, saugfähigen Materialabschnittes entsprechend kleiner ist

Weiterhin ist der Absorptionskörper bevorzugt Bindemittelfrei ausgelegt.

Der beschriebene Absorptionskörper kann bei chronischen Wunden, die ggf. eine leichte Blutung aufweisen, wie sie z.B. bei Ulcus cruris, Dekubitus oder ähnlichen Erkrankungen auftreten, ist in unmittelbaren Kontakt mit der Wunde gebracht werden. Hier ist die Gefahr eines kritischen Blutverlustes nicht zu befurchten. Es steht hier einerseits die Aufnahme von Exsudaten sowie die Einschränkung der Restblutung bzw. der Blutungsneigung durch dass bzw. die betreffenden Agenzien im Vordergrund. Anfänglich weiter austretendes Blut soll hier überdies durch den Superabsorber der Wundauflage aufgenommen werden. Der besagte mindestens eine chemisch und/oder pharmakologisch und/oder physiologisch wirkende Wirkstoff bzw. Wirkstoff komplex kann dabei in dem der Wunde zugewandten Bereich der Wundauflage angeordnet sein. Bei akut blutenden Wunden, wie sie z.B. durch ein Akuttrauma hervorgerufen werden, kann hingegen vorgesehen sein, dass
c) der Absorptionskörper als Druck- oder Kompressionsverband ausgebildet und/oder in einen solchen integrierbar ist, und/oder
d) der Absorptionskörper stromabwärts einer arteriellen Abbindung angeordnet ist.

In diesen Fällen liegt die Hauptlast der Blutstillung bei dem Druck- oder Kompressionsverband bzw. bei der Abbindung. Bei beiden handelt es sich also um physikalisch wirkende Wirkelemente im obigen Sinne. Dem Wundpflegeartikel obliegt dabei die Aufgabe, anfänglich weiter austretendes Blut durch den Superabsorber aufzunehmen.

Fig. 2 zeigt einen Druckverband 20, der um ein Körperteil 21 aufweisend eine stark blutende Wunde 22 angelegt ist. der Druckverband besteht aus einer primären Wundauflage 23 sowie einem nicht zu harten, nicht saugfähigen Druckpolster 24 ohne scharfe oder harte Kanten, der mithilfe einer Wickel 26 mit mäßigem Zug befestigt wird. Bei dem Druckpolster 24 kann es sich z.B. um ein flüssigkeitsdicht verpacktes Kissen aus Zellstoff handeln, ebenso aber z.B. um einen Knebel oder einen kugelförmigen Gegenstand. Durch den ausgeübten Druck wird die Durchblutung des betreffenden Körperteils gemindert und die traumatisch geöffneten Blutgefäße werden wieder geschlossen. Weiterhin ist zwischen Wickel 26 und Druckpolster 24 ein erfindungsgemäßer Wundpflegeartikel in Form einer sekundären Wundauflage 25 enthaltend superabsorbierende Polymere angeordnet. Dem Wundpflegeartikel 25 obliegt dabei die Aufgabe, trotz des Druckverbands weiter aus der Wunde 22 austretendes Blut durch den Superabsorber aufzunehmen. In dieser Ausgestaltung ist überdies vorteilhaft, dass

etwaige durchsickernde Flüssigkeit, die von den Superabsorbierenden Polymeren aufgenommen wird, zu einem Aufquellen letzterer führt, wodurch wiederum der auf die Wunde ausgeübte physikalische Druck erhöht wird. Die primäre Wundauflage 23 kann überdies mindestens einen chemisch und/oder pharmakologisch und/oder physiologisch wirkenden Wirkstoffbzw. Wirkstoffkomplex aufweisen.

Fig. 3 zeigt einen erfindungsgemäßen Wundpflegeartikel 30 der ähnlich wie ein herkömmliches Wundverbandpäckchen aufgebaut ist, aufweisend eine primäre Wundauflage 31 sowie eine Verbandwickel 32, die mindestens abschnittsweise mit einem superabsorbierenden Polymer 33 versehen ist. Letzterem obliegt dabei die Aufgabe, trotz des Druckverbands weiter aus der Wunde austretendes Blut aufzunehmen. Der blutstillende Effekt dieses Wundpflegeartikels kann durch mindestens einen in der primären Wundauflage 31 angeordneten chemisch und/oder pharmakologisch und/oder physiologisch wirkenden Wirkstoff bzw. Wirkstoffkomplex hervorgerufen werden. Alternativ oder zusätzlich dazu kann der Wundpflegeartikel wie ein Druckverband verwendet werden, dabei wirkt die primäre Wundauflage 31 als Druckpolster, oder es kann ein gesondertes Druckpolster verwendet werden. In diesem Falle ist handelt es sich also bei dem blutstillenden Agenz um ein physikalisch wirkendes Wirkelement. Auch in dieser Ausgestaltung führt etwaige durchsickernde Flüssigkeit, die von den Superabsorbierenden Polymeren aufgenommen wird, zu einem Aufquellen letzterer führt, wodurch wiederum der auf die Wunde ausgeübte physikalische Druck erhöht wird

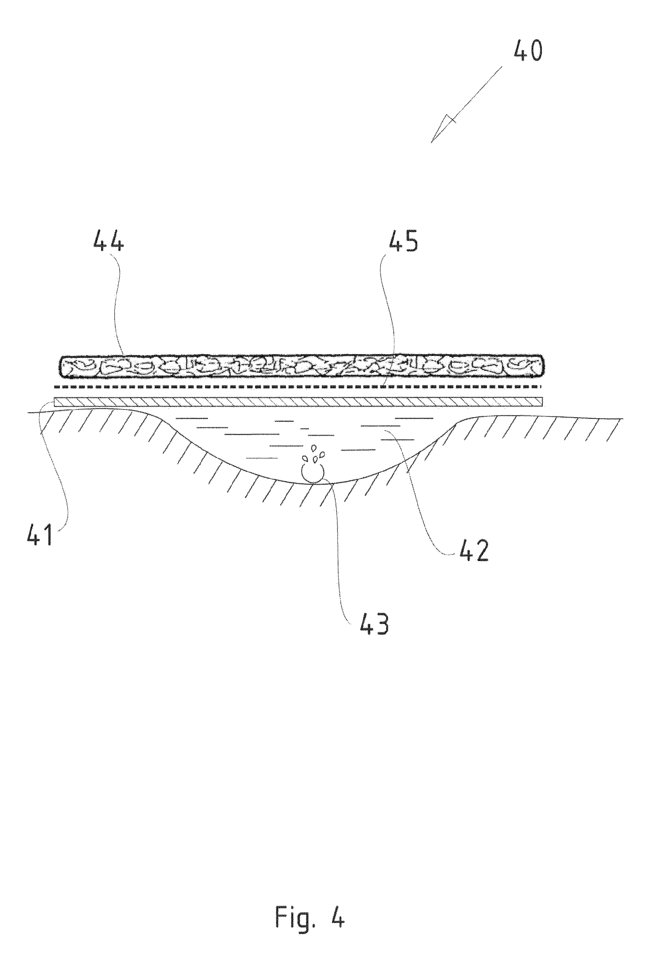

Fig. 4 zeigt ebenfalls einen erfindungsgemäßen Wundpflegeartikel 40 bestehend aus einer primären Wundauflage 41, die mindestens einen chemisch und/oder pharmakologisch und/oder physiologisch wirkenden Wirkstoff bzw. Wirkstoffkomplex aufweist. Diese primäre Wundauflage 41 wird unmittelbar auf eine Wunde 42 aufgelegt, die eine stark blutende Verletzung eines Gefäßes 43 aufweist. Die primäre Wundauflage wirkt nicht oder nur unwesentlich absorbierend. Peripher von dieser primären Wundauflage ist eine sekundäre

Wundauflage 44 angeordnet, die z.B. der in Fig. 1 dargestellten Wundauflage entspricht und superabsorbierende Polymere enthält, im Unterschied zu letzterer jedoch keine Hülle aufweist (aber aufweisen kann). Zwischen primärer und sekundärer Wundauflage ist eine Diffusionsbarriere 45 angeordnet, die so ausgestaltet sein, dass Blut bzw. Serumflüssigkeit erst ab einem bestimmten hydrostatischen Druck durch die Barriere oder dass sie Poren aufweist, deren Durchmesser geringer ist als der der Blutzellen. Diese Diffusionsbarriere kann beispielsweise aus modifizierter Cellulose (bevorzugt CMC) bestehen. Auf diese Weise wird verhindert, dass der in der sekundären Wundauflage angeordnete Superabsorber eine Sogwirkung auf die Wunde ausübt und so die Blutung forciert. Um das Ensemble aus primärer Wundauflage, Diffusionsbarriere und sekundärer Wundauflage kann z.B. eine in Fig. 4 nicht dargestellte Wickel angeordnet werden, um die drei Bestandteile an Ort und Stelle zu immobilisieren. Ebenso können die drei Bestandteile auch in einem nicht dargestellten kompakten, einheitlichen Produkt vereint sein. Hierzu können sie z.B. in einer gemeinsamen Hülle angeordnet sein und/oder mit einer verbunden sein, z.B. durch Thermische Schweißung, Ultraschallnähte, physiologische Klebstoffe wie Stärke- oder Eiweißkleber und dergleichen. Ein solches Produkt kann z.B mit Pflastern oder einer Wickel an Ort und Stelle immobilisiert werden.

Die gezeigte Diffusionsbarriere 45 kann in einigen Fällen verzichtbar sein, z.B. dann wenn die verwendeten Superabsorber so ausgestaltet sind, dass sie keine Blutzellen aufnehmen, sondern lediglich Blutserumsflüssigkeit.

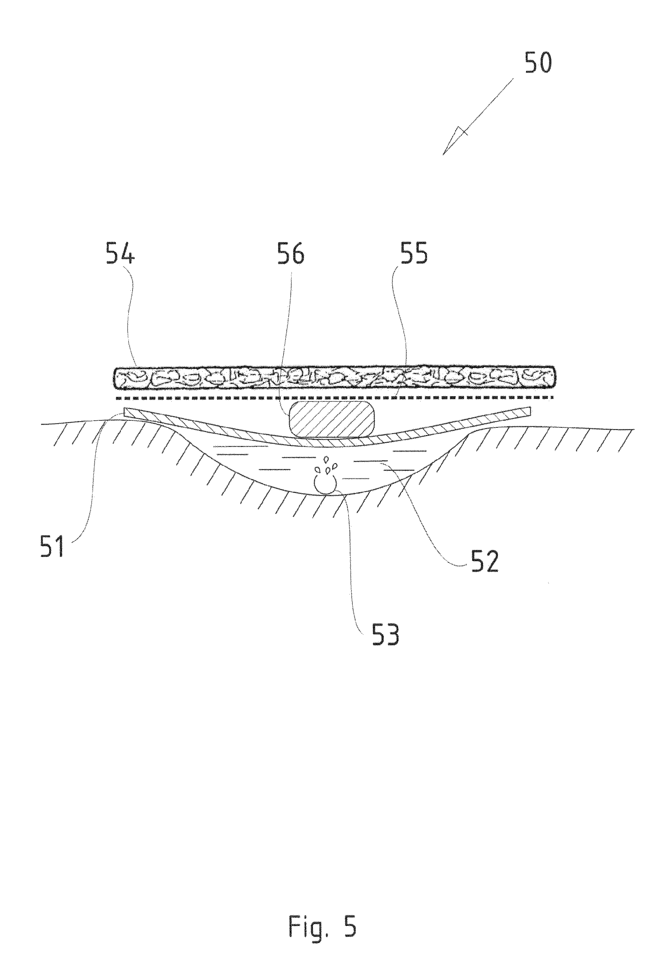

Fig. 5 zeigt eine ähnliche Ausgestaltung wie Fig. 4. Allerdings ist hier zusätzlich noch ein Druckpolster 56 vorgesehen, dass zwischen der primären Wundauflage 51 und der Diffusionsbarriere 55 angeordnet ist, es kann jedoch auch zwischen Druckpolster 56 und sekundärer Wundauflage 54 angeordnet sein. Die Diffusionsbarriere kann beispielsweise aus modifizierter Cellulose (bevorzugt CMC) bestehen.

Bei dem Druckpolster kann es sich z.B. um ein flüssigkeitsdicht verpacktes Kissen aus Zellstoff handeln, ebenso aber z.B. um einen Knebel oder einen kugelförmigen Gegenstand. Im Unterschied zu der in Fig. 4 gezeigten Konfiguration ist in dieser Konfiguration neben bzw. anstelle des chemisch und/oder pharmakologisch und/oder physiologisch wirkenden Wirkstoffs bzw. Wirkstoffkomplexes ein physikalisch wirkendes Wirkelement als blutstillendes Agenz verwirklicht.

## Patentansprüche

1. Wundpflegeartikel, aufweisend eine Menge an superabsorbierenden Polymeren, **dadurch gekennzeichnet, dass** der Wundpflegeartikel mindestens ein Agenz aufweist, das die Blutung oder die Blutungsneigung einschränken kann.

2. Wundpflegeartikel gemäß Anspruch 1, **dadurch gekennzeichnet, dass** es sich bei dem Agenz um mindestens einen chemisch und/oder pharmakologisch und/oder physiologisch wirkenden Wirkstoff bzw. Wirkstoffkomplex handelt.

3. Wundpflegeartikel gemäß einem der Ansprüche 1-2, **dadurch gekennzeichnet, dass** es sich bei dem Agenz um mindestens ein physikalisch wirkendes Wirkelement handelt.

4. Wundpflegeartikel gemäß einem der vorherigen Ansprüche, **dadurch gekennzeichnet, dass** der Wundpflegeartikel
a) als im wesentlichen flacher Materialabschnitt aufweisend Absorptionsmaterial vorliegt, der aus einem Vlies mit darin verteilten superabsorbierenden Polymeren sowie mindestens einem chemisch und/oder pharmakologisch und/oder physiologisch wirkenden Wirkstoff bzw. Wirkstoffkomplex ausgebildet ist;
b) als oder in Kombination mit einem Druck- oder Kompressionsverband ausgebildet ist;
c) als eine Kombination aus einer primären, nicht oder nur unwesentlich absorbierenden Wundauflage, die mindestens einen chemisch und/oder pharmakologisch und/oder physiologisch wirkenden Wirkstoff bzw. Wirkstoffkomplex aufweist, und einer peripher von dieser primären Wundauflage angeordneten sekundären Wundauflage, die superabsorbierende Polymere enthält, ausgebildet ist, wobei ggf. zwischen beiden eine Diffusionsbarriere angeordnet ist,
d) in Form eines Verbandpäckchens, aufweisend eine primäre Wundauflage mit mindestens einem chemisch und/oder pharmakologisch und/oder physiologisch wirkenden Wirkstoff bzw. Wirkstoff komplex sowie einem an der Wundauflage angeordneten Wickelabschnitt, der zumindest abschnittsweise superabsorbierende Polymere aufweist, ausgebildet ist und/oder
e) als Materialabschnitt mit einer Längserstreckung aufweisend Absorptionsmaterial, wobei der Materialabschnitt elastisch verformbare Eigenschaften aufweist, und wobei der Materialabschnitt superabsorbierende Polymere sowie ggf. mindestens einen chemisch und/oder pharmakologisch und/oder physiologisch wirkende Wirkstoff bzw. Wirkstoff komplex aufweist, ausgebildet ist.

5. Wundpflegeartikel gemäß einem der vorherigen Ansprüche, **dadurch gekennzeichnet, dass** der Wundpflegeartikel außerdem mindestens einen nutritiven, mindestens einen desinfizierenden bzw. dekontaminierenden und/oder mindestens einen Proteasen hemmend wirkenden Wirkstoff und/oder Wirkstoff komplex aufweist.

6. Verwendung eines Wundpflegeartikels gemäß einem der vorherigen Ansprüche für chronische Wunden, die ggf. eine leichte Blutung aufweisen, wobei die Wundauflage in unmittelbaren Kontakt mit der Wunde gebracht wird.

7. Verwendung eines Wundpflegeartikels gemäß einem der vorherigen Ansprüche für akut blutende Wunden, wobei der Wundpflegeartikel als Druck- oder Kompressionsverband ausgebildet und/oder in ein solchen integrierbar ist.

8. Verwendung eines Wundpflegeartikels gemäß einem der vorherigen Ansprüche für akut blutende Wunden, wobei der Wundpflegeartikel stromabwärts einer arteriellen Abbindung angeordnet ist.

9. Verwendung eines Wundpflegeartikels gemäß einem der vorherigen Ansprüche für traumatisch erzeugte Wunden.

10. Verwendung eines Wundpflegeartikels gemäß einem der vorherigen Ansprüche für militärische Zwecke.

11. Kit für die Akut-, Notfall- oder Militärmedizinische bzw. die chronische Versorgung, aufweisend einen Wundpflegeartikel gemäß einem der vorherigen Ansprüche.

12. Verwendung eines Wundpflegeartikels gemäß einem der vorherigen Ansprüche zur Behandlung von Wunden, die aufgrund einer Erkrankung wie z.B. einer Hämophilie und/oder eines systemisch, pathologisch und/oder pharmakologisch hervorgerufenen Gerinnungsdefizits einen verschlechterten Heilungsverlauf aufweisen.

13. Verwendung eines Wundpflegeartikels gemäß einem der vorherigen Ansprüche zur operativen bzw. postoperativen Versorgung.
